(19) Europäisches Patentamt
European Patent Office
Office européen des brevets

(11) **EP 3 457 115 A1**

(12) **EUROPÄISCHE PATENTANMELDUNG**

(43) Veröffentlichungstag:
20.03.2019  Patentblatt 2019/12

(21) Anmeldenummer: 18193917.4

(22) Anmeldetag: **12.09.2018**

(51) Int Cl.:
$G01N\ 21/3504^{(2014.01)}$ $A61L\ 2/07^{(2006.01)}$
$A61L\ 2/28^{(2006.01)}$ $G01N\ 21/31^{(2006.01)}$

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **15.09.2017  DE 102017121440**

(71) Anmelder: **Miele & Cie. KG**
**33332 Gütersloh (DE)**

(72) Erfinder:
• **Gregor, Olaf**
**83373 Taching (DE)**
• **Hillmann, Johannes**
**89129 Langenau (DE)**
• **van Doornmalen, Josephus**
**5688 Oirschot (NL)**
• **Öhlinger, Andreas**
**5110 Oberndorf (AT)**
• **Sevcik, Roland**
**83435 Bad Reichenhall (DE)**
• **Eder, Horst**
**5120 St. Pantaleon (AT)**
• **Ismic, Dzenan**
**5110 Oberndorf bei Salzburg (AT)**
• **Kopinga, Klaas**
**5506 Veldhoven (NL)**

(54) **VORRICHTUNG ZUM ERFASSEN UND VERFAHREN ZUR ERMITTLUNG EINER DAMPFKONZENTRATION IN EINEM STERILISATOR SOWIE STERILISATORVORRICHTUNG**

(57)    Die Erfindung betrifft eine Vorrichtung (104) zum Erfassen einer Dampfkonzentration in einem Sterilisator mit einem einen Sterilisationsraum (106) umschließenden Doppelmantel (108), wobei der Doppelmantel (108) eine innere Wand (110) und eine äußere Wand (112) mit einer Mantelöffnung aufweist. Die Vorrichtung (104) umfasst ein Gehäuse, das mit der äußeren Wand (112) des Sterilisators (102) koppelbar ist und eine Gehäuseöffnung aufweist, über die ein von dem Gehäuse umschlossener Hohlraum (454) fluidisch mit der Mantelöffnung verbindbar ist, eine Messkammer (456), die in dem Hohlraum (454) angeordnet ist, eine Verbindungsleitung (118), die mit der Messkammer (456) gekoppelt ist, um die Messkammer (456) fluidisch mit einem Sterilisationsraum (106) des Sterilisators zu verbinden, eine erste Lichtleiteinrichtung (340) zum Einleiten zumindest eines ersten elektromagnetischen Strahls in ein von der Messkammer (456) umfasstes Messlumen, und eine zweite Lichtleiteinrichtung (342) zum Ausleiten zumindest eines Anteils des zumindest einen ersten elektromagnetischen Strahls aus dem Messlumen.

FIG 4

**Beschreibung**

Die Erfindung betrifft eine Vorrichtung zum Erfassen und ein Verfahren zur Ermittlung einer Dampfkonzentration in einem Sterilisator sowie eine Sterilisatorvorrichtung

Eine wichtige Sterilisationsbedingung während eines Sterilisationsprozesses ist das Vorhandensein von Sattdampf.

Der Erfindung stellt sich die Aufgabe, eine verbesserte Vorrichtung zum Erfassen und ein verbessertes Verfahren zur Ermittlung einer Dampfkonzentration in einem Sterilisator sowie eine verbesserte Sterilisatorvorrichtung zu schaffen.

Erfindungsgemäß wird diese Aufgabe durch eine Vorrichtung zum Erfassen und ein Verfahren zur Ermittlung einer Dampfkonzentration in einem Sterilisator sowie eine Sterilisatorvorrichtung mit den Merkmalen der Hauptansprüche gelöst. Vorteilhafte Ausgestaltungen und Weiterbildungen der Erfindung ergeben sich aus den nachfolgenden Unteransprüchen.

Ein mit der Erfindung erreichbare Vorteil besteht darin, dass das Vorhandensein von Sattdampf während eines Sterilisationsprozesses erkannt werden kann.

Eine Vorrichtung zum Erfassen einer Dampfkonzentration in einem Sterilisator mit einem einen Sterilisationsraum umschließenden Doppelmantel, wobei der Doppelmantel eine innere Wand und eine äußere Wand mit einer Mantelöffnung aufweist, weist die folgenden Merkmale auf:

ein Gehäuse, das mit der äußeren Wand des Sterilisators koppelbar ist und eine Gehäuseöffnung aufweist, über die ein von dem Gehäuse umschlossener Hohlraum fluidisch mit der Mantelöffnung verbindbar ist;

eine Messkammer, die in dem Hohlraum angeordnet ist;

eine Verbindungsleitung, die mit der Messkammer gekoppelt ist, um die Messkammer fluidisch mit einem Sterilisationsraum des Sterilisators zu verbinden;

eine erste Lichtleiteinrichtung zum Einleiten zumindest eines ersten elektromagnetischen Strahls in ein von der Messkammer umfasstes Messlumen; und

eine zweite Lichtleiteinrichtung zum Ausleiten zumindest eines Anteils des zumindest einen ersten elektromagnetischen Strahls aus dem Messlumen.

Bei dem Sterilisator kann es sich um ein Gerät zum Sterilisieren von aufzubereitenden Gütern, beispielsweise von chirurgischen Instrumenten handeln. Der Sterilisator kann als Dampf-Sterilisator, beispielsweise als Dampf-Groß-Sterilisator oder Klein-Sterilisator ausgeführt sein. Die aufzubereitenden Güter können während des Betriebs des Sterilisators in dem Sterilisationsraum angeordnet sein. Der Doppelmantel kann den Sterilisationsraum voll oder teilweise umschließen. Der Sterilisationsraum kann während des Betriebs des Sterilisators mit Dampf, beispielsweise Wasserdampf, befüllt sein. Zwischen der inneren Wand und der äußeren Wand kann während des Betriebs des Sterilisators Dampf strömen. Die Mantelöffnung kann als Durchgangsöffnung in der äußeren Wand ausgeführt sein. Die Vorrichtung kann als Sensor oder Teil eines Sensors zur Ermittlung der Dampfkonzentration ausgeführt sein. Das Gehäuse der Vorrichtung kann direkt auf eine äußere Seite der äußeren Wand aufgesetzt werden oder über ein Zwischenstück mit der äußeren Wand verbunden werden. Die Gehäuseöffnung kann korrespondierend zu der Mantelöffnung ausgeführt sein. Über die Mantelöffnung und die Gehäuseöffnung kann durch den Doppelmantel strömender Dampf in den Hohlraum der Vorrichtung einströmen. Dadurch kann die in dem Hohlraum angeordnete Messkammer beheizt werden. Zudem kann aus dem Hohlraum Dampf zum Kalibrieren der Vorrichtung entnommen werden. Die Verbindungsleitung kann bei an dem Sterilisator montierter Vorrichtung durch den Doppelmantel in den Sterilisationsraum führen, so dass sich eine in der Messkammer befindliche Dampfkonzentration an die sich in dem Sterilisationsraum befindliche Dampfkonzentration anpassen kann. Eine Lichtleiteinrichtung kann gemäß unterschiedlicher Ausführungsformen beispielsweise als Lichtleiter, lichtdurchlässiges Element oder Lichtleitkanal ausgeführt sein. Unter einem elektromagnetischen Strahl kann beispielsweise ein Lichtstrahl verstanden werden. Die erste Lichtleiteinrichtung kann mit einer Sendeeinrichtung zum Aussenden des zumindest einen Strahl gekoppelt sein. Der zumindest eine Strahl kann nach Passieren der Messkammer oder eines Abschnitts der Messkammer von der zweiten Lichtleiteinrichtung aufgenommen werden. Die zweite Lichtleiteinrichtung kann mit einer Empfangseinrichtung gekoppelt sein. Beispielsweise kann von der Empfangseinrichtung eine Intensität des zumindest einen Strahls erfasst und als Intensitätswert für eine weitere Auswertung oder Verarbeitung bereitgestellt werden. Gemäß einer Ausführungsform ist die erste Lichtleiteinrichtung ausgebildet, um zusätzlich zu dem ersten elektromagnetischen Strahl zumindest einen zweiten elektromagnetischen Strahl in das von der Messkammer umfasste Messlumen einzuleiten. Dabei können die beiden Strahlen durch Dampf unterschiedlich gedämpft werden, beispielsweise aufgrund unterschiedlicher Wellenlängen.

Das Gehäuse kann einen die Gehäuseöffnung umschließenden Gehäuseflansch zum mechanischen Verbinden des Gehäuses mit der äußeren Wand des Doppelmantels aufweisen. Beispielsweise kann der Gehäuseflansch ausgeformt sein, um auf einen an der äußeren Wand des Doppelmantels ausgeformten Mantelflansch aufgesetzt zu werden. Der Gehäuseflansch kann zur fluiddichten Befestigung des Gehäuses an der äußeren Wand des Doppelmantels verwendet werden.

Die Vorrichtung kann ein Dampfventil aufweisen. Das Dampfventil kann ausgangsseitig mit einer Dampfventilöffnung der Messkammer verbunden und ausgebildet sein, um Kalibrierdampf in die Messkammer einzulassen. Dadurch ist eine einfache Kalibrierung möglich, auch wenn die Vorrichtung am Sterilisator verbaut ist.

Beispielsweise kann das Dampfventil eingangsseitig mit dem Hohlraum verbunden sein. Auf diese Weise kann der sich in dem Doppelmantel befindliche Dampf als Kalibrierdampf verwendet werden. Alternativ kann der Kalibrierdampf von extern zugeführt werden.

Die Vorrichtung kann ein Belüftungsventil aufweisen, das ausgangsseitig mit einer Belüftungsöffnung der Messkammer verbunden ist, und ausgebildet ist, um Reinluft in die Messkammer einzulassen. Die Reinluft kann zum Konditionieren der Vorrichtung verwendet werden.

Die erste Lichtleiteinrichtung kann zumindest einen durch das Gehäuse zu einer ersten Lichtleiteröffnung der Messkammer führenden ersten Lichtleiter umfassen. Auf diese Weise kann eine Sendeeinrichtung zum Aussenden des Lichtstrahls außerhalb des Gehäuses angeordnet werden. Zusätzlich oder alternativ kann die zweite Lichtleiteinrichtung zumindest einen durch das Gehäuse zu einer zweiten Lichtleiteröffnung der Messkammer führenden zweiten Lichtleiter aufweisen. Auf diese Weise kann eine Empfangseinrichtung zum Empfangen des Lichtstrahls außerhalb des Gehäuses angeordnet werden.

Die Vorrichtung kann eine Sendeeinrichtung umfassen, die ausgebildet ist, um den eine erste Wellenlänge aufweisenden ersten elektromagnetischen Strahl bereitzustellen und in die die erste Lichtleiteinrichtung einzukoppeln. Die Sendeeinrichtung kann außerhalb des Gehäuses, innerhalb des Gehäuse oder gemäß einer Ausführungsform innerhalb der Messkammer angeordnet sein. Zusätzlich oder alternativ kann die Sendeeinrichtung ausgebildet sein, um einen zweiten elektromagnetischen Strahl bereitzustellen und in die erste Lichtleiteinrichtung einzukoppeln. Der zweite elektromagnetische Strahl kann eine sich von der ersten Wellenlänge unterscheidende zweite Wellenlänge aufweisen.

Beispielsweise kann die Sendeeinrichtung eine erste Leuchtdiode zum Aussenden des ersten elektromagnetischen Strahls und gegebenenfalls eine zweite Leuchtdiode zum Aussenden des zweiten elektromagnetischen Strahls aufweisen. Die beiden Leuchtdioden können als separate Bauteile ausgeführt sein oder in ein Bauteil integriert sein, beispielsweise als sogenannte Doppel-LED.

Die Vorrichtung kann eine Empfangseinrichtung aufweisen, die mit der zweiten Lichtleiteinrichtung gekoppelt sein kann. Die Empfangseinrichtung kann ausgebildet sein, um ein eine Intensität des zumindest einen Anteils des ersten elektromagnetischen Strahls repräsentierendes erstes Intensitätssignal bereitzustellen. Die Empfangseinrichtung kann außerhalb des Gehäuses, innerhalb des Gehäuse oder gemäß einer Ausführungsform innerhalb der Messkammer angeordnet sein. Zusätzlich oder alternativ kann die Empfangseinrichtung ausgebildet sein, um ein eine Intensität zumindest eines Anteils eines zweiten elektromagnetischen Strahls repräsentierendes zweites Intensitätssignal bereitzustellen.

Die Vorrichtung kann eine Ermittlungseinrichtung aufweisen, die ausgebildet sein kann, um unter Verwendung des ersten Intensitätssignals und des zweiten Intensitätssignals einen die Dampfkonzentration repräsentierenden Wert zu ermitteln. Die Ermittlungseinrichtung kann beispielsweise als integrierte Schaltung sehr kostengünstig ausgeführt sein.

Eine Sterilisatorvorrichtung weist die folgenden Merkmale auf:

einen Sterilisator mit einem einen Sterilisationsraum umschließenden Doppelmantel, wobei der Doppelmantel eine innere Wand und eine äußere Wand mit einer Mantelöffnung aufweist; und

eine genannte Vorrichtung zum Erfassen einer Dampfkonzentration in dem Sterilisator.

Somit kann die genannte Vorrichtung vorteilhaft im Zusammenhang mit einem Sterilisator eingesetzt werden.

Bei der Sterilisatorvorrichtung kann der Doppelmantel im Betrieb der Sterilisatorvorrichtung von Dampf durchströmt werden. Dadurch kann eine Temperatur in dem Sterilisationsraum eingestellt werden.

Ein Verfahren zur Kalibrierung einer genannten Vorrichtung zum Erfassen einer Dampfkonzentration umfasst die folgenden Schritte:

Einlassen von Reinluft in die Messkammer;

Bestimmen eines Nullpunktwerts unter Verwendung einer Intensität des zumindest einen ersten Strahls nach Einleitung des ersten Strahls in die die Reinluft umfassende Messkammer;

Einlassen von Kalibrierdampf in die Messkammer; und

Bestimmen eines Kalibrierwertes unter Verwendung einer Intensität des zumindest einen ersten Strahls nach Einleitung des ersten Strahls in die den Kalibrierdampf umfassende Messkammer.

Der Nullpunktwert kann eine Intensität des zumindest einen ersten Strahls bei Nichtvorhandensein von Dampf repräsentieren. Der Kalibrierwert kann eine Intensität des Anteils des zumindest einen ersten Strahls bei Vorhandensein von Dampf repräsentieren. Somit kann es sich bei den beiden Werten um Referenzwerte handeln, die anschließen in die Ermittlung der Dampfkonzentration einfließen können.

Ein Verfahren zur Ermittlung einer Dampfkonzentration in einem Sterilisator unter Verwendung einer genannten Vorrichtung zum Erfassen einer Dampfkonzentration umfasst die folgenden Schritte:

Bestimmen einer Intensität des zumindest einen Anteils des zumindest einen ersten Strahls, während die Messkammer über die Verbindungsleitung fluidisch mit dem Sterilisationsraum verbunden ist; und

Bestimmen eines die Dampfkonzentration repräsentierenden Werts unter Verwendung der Intensität, eines Nullpunktwerts und eines Kalibrierwertes, wobei der Nullpunktwert und der Kalibrierwert gemäß dem genannten Verfahren zur Kalibrierung bestimmte Werte repräsentieren.

Der hier vorgestellte Ansatz schafft ferner eine Steuervorrichtung, die ausgebildet ist, um die Schritte einer Variante eines hier vorgestellten Verfahrens in entsprechenden Einrichtungen durchzuführen, anzusteuern bzw. umzusetzen. Auch durch diese Ausführungsvariante der Erfindung in Form einer Steuervorrichtung kann die der Erfindung zugrunde liegende Aufgabe schnell und effizient gelöst werden.

Die Steuervorrichtung kann ausgebildet sein, um Eingangssignale einzulesen und unter Verwendung der Eingangssignale Ausgangssignale zu bestimmen und bereitzustellen. Ein Eingangssignal kann beispielsweise ein über eine Eingangsschnittstelle der Steuervorrichtung einlesbares Sensorsignal darstellen. Ein Ausgangssignal kann ein Steuersignal oder ein Datensignal darstellen, das an einer Ausgangsschnittstelle der Steuervorrichtung bereitgestellt werden kann. Die Steuervorrichtung kann ausgebildet sein, um die Ausgangssignale unter Verwendung einer in Hardware oder Software umgesetzten Verarbeitungsvorschrift zu bestimmen. Beispielsweise kann die Steuervorrichtung dazu eine Logikschaltung, einen integrierten Schaltkreis oder ein Softwaremodul umfassen und beispielsweise als ein diskretes Bauelement realisiert sein oder von einem diskreten Bauelement umfasst sein.

Der beschriebene Ansatz ermöglicht die Realisierung eines Verfahrens und eines Sensors zur Ermittlung von Dampfkonzentration inklusive Konditionierung und Kalibrierung. Dabei kann der Sensor als ein sogenannter 4D-Sensor ausgeführt werden. Ein entsprechender Sensor, beispielsweise für einen Dampf-Groß-Sterilisator, kann neben den klassischen Sterilisationsparametern Druck, Temperatur und Zeit auch das Vorhandensein von Sattdampf als zusätzliche Sterilisationsbedingung innerhalb eines Sterilisationszyklus überwachen und die sensierten Informationen beispielsweise an die Software des Sterilisators zur weiteren Analyse weiterleiten. Anhand der nachfolgenden Figuren werden technische Lösungen sowie mögliche Implementationsweisen zur mechanischen Anbindung eines solchen Sensors an einen Sterilisator, die Messmethode, das Messverfahren, die Signalaufbereitung und der Datentransfer zur Sterilisatorsoftware beschrieben.

Ein Ausführungsbeispiel der Erfindung ist in den Zeichnungen rein schematisch dargestellt und wird nachfolgend näher beschrieben. Es zeigt

Figur 1      eine Sterilisatorvorrichtung gemäß einem Ausführungsbeispiel;

Figur 2      eine Vorrichtung zum Erfassen einer Dampfkonzentration gemäß einem Ausführungsbeispiel;

Figur 3      eine Vorrichtung zum Erfassen einer Dampfkonzentration gemäß einem Ausführungsbeispiel;

Figur 4      eine Vorrichtung zum Erfassen einer Dampfkonzentration gemäß einem Ausführungsbeispiel;

Figur 5      eine Vorrichtung zum Erfassen einer Dampfkonzentration gemäß einem Ausführungsbeispiel;

Figur 6      eine Vorrichtung zum Erfassen einer Dampfkonzentration gemäß einem Ausführungsbeispiel;

Figur 7      eine Sendeeinrichtung gemäß einem Ausführungsbeispiel;

Figur 8      eine Sendeeinrichtung gemäß einem Ausführungsbeispiel;

Figur 9      ein Ablaufdiagramm eines Verfahrens zur Kalibrierung einer Vorrichtung zum Erfassen einer Dampfkonzentration gemäß einem Ausführungsbeispiel;

Figur 10      ein Ablaufdiagramm eines Verfahrens zur Ermittlung einer Dampfkonzentration in einem Sterilisator unter Verwendung einer Vorrichtung zum Erfassen einer Dampfkonzentration gemäß einem Ausführungsbeispiel;

Figur 11      ein Ablaufdiagramm eines Verfahrens zur Konditionierung einer Vorrichtung zum Erfassen einer Dampfkonzentration gemäß einem Ausführungsbeispiel; und

Figur 12      ein Ablaufdiagramm eines Verfahrens zur Kalibrierung einer Vorrichtung zum Erfassen einer Dampfkonzentration gemäß einem Ausführungsbeispiel.

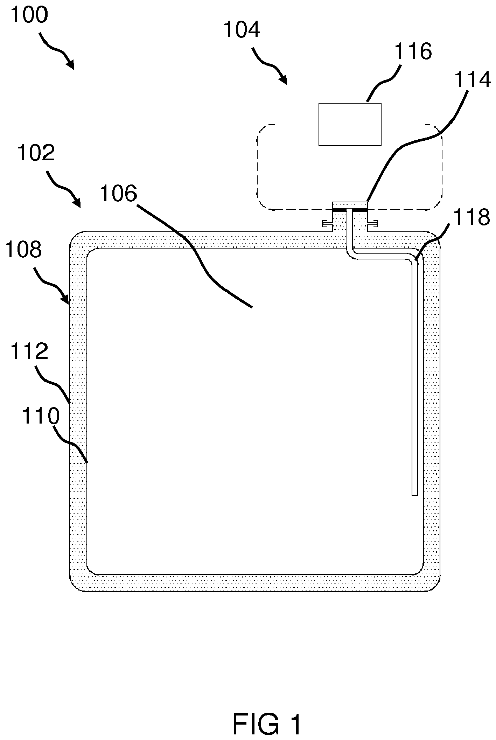

Figur 1 zeigt eine Sterilisatorvorrichtung 100 gemäß einem Ausführungsbeispiel. Die Sterilisatorvorrichtung 100 umfasst einen Sterilisator 102 und eine Vorrichtung 104 zum Erfassen einer Dampfkonzentration in einem Sterilisationsraum 106 des Sterilisators 102. Der Sterilisator 102 weist einen Doppelmantel 108 auf, der den Sterilisationsraum 106 umschließt. Der Doppelmantel 108 weist eine innere Wand 110 und eine äußere Wand 112 auf. Der Doppelmantel 108 kann im Betrieb der Sterilisatorvorrichtung 100 von Dampf durchströmt werden. Innerhalb des Sterilisationsraums 106 können zu sterilisierende Güter angeordnet werden. Die Vorrichtung 104 zum Erfassen der Dampfkonzentration in dem Sterilisationsraum 106 ist an einer Außenseite des Sterilisators 102 angeordnet. Die Vorrichtung 104 ist über eine Verbindungsleitung 118 der Vorrichtung 104 mit dem Sterilisationsraum 106 gekoppelt.

Der Sterilisationsraum 106 kann auch als Sterilisatorkammer und die Vorrichtung 104 auch als Sensor bezeichnet werden. Gemäß einem Ausführungsbeispiel ist die Vorrichtung 104 als ein 4D-Sensor ausgeführt.

Die Vorrichtung 104 umfasst einen Sensorkopf 114. Optional umfasst die Vorrichtung 104 ferner eine Betriebseinrichtung 116, die beispielsweise eine Optik und zusätzlich oder alternativ eine Elektronik umfasst.

Gemäß einem Ausführungsbeispiel ermöglicht es die Vorrichtung 104, das neben dem reinen Monitoring von Signalen ein die Dampfkonzentration im Sterilisationsraum 106 repräsentierendes Signal zur Regelung und/oder Steuerung eines Sterilisationsprozesses zu verwenden. Führende Parameter zur Prozesssteuerung sind dann nicht mehr Druck bzw. Temperatur und Zeit sondern die Signale des hier beschriebenen Sensors.

Dies ermöglicht die Verwendung der Dampfkonzentration als einen alternativen oder weiteren Parameter zur Validierung von Sterilisationsbedingungen, um die Sterilisationsbedingungen präziser, wichtig für die Patientensicherheit, und umfassender zu ermitteln, zu dokumentieren und/oder die Steuerung eines Sterilisationsprozesses optimieren zu können. Speziell in Verbindung mit immer kleineren, lumigen Operationswerkzeugen der minimalinvasiven Chirurgie als aufzubereitende Güter ist dies vorteilhaft.

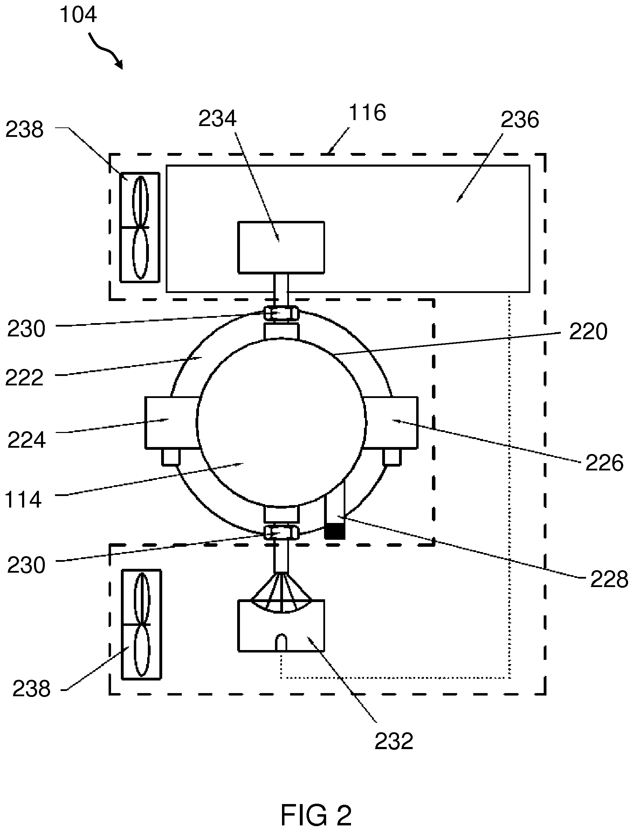

Figur 2 zeigt eine Vorrichtung 104 zum Erfassen einer Dampfkonzentration gemäß einem Ausführungsbeispiel. Dabei kann es sich um ein Ausführungsbeispiel der anhand von Figur 1 beschriebenen Vorrichtung handeln. Die Vorrichtung 104 umfasst den Sensorkopf 114 und die Betriebseinrichtung 116.

Der Sensorkopf 114 weist ein Gehäuse 220 auf, das eine Isolierung 222 aufweist und an dem Doppelmantel des Sterilisators befestigt werden kann. Ferner weist der Sensorkopf 114 ein Dampfventil 224, ein Belüftungsventil 226, eine Belüftung 228 mit Reinluftfilter sowie zwei Lichtfaserdurchführungen 230 auf.

Die Betriebseinrichtung 116 ist gemäß diesem Ausführungsbeispiel von einem Gehäuse umschlossen und umfasst eine Sendeeinrichtung 232, die beispielhaft als eine Optik mit LEDs ausgeführt ist, eine Empfangseinrichtung 234 und eine Leiterplatte 236 mit einer Elektronik. Ferner ist eine aktive Kühlung 238 vorgesehen.

Gemäß einem Ausführungsbeispiel ist auf der Leiterplatte 236 eine Steuervorrichtung zum Steuern eines Betriebs der Sendeeinrichtung 232 und optional zusätzlich zum Betrieb der Ventile 224, 226 angeordnet. Gemäß einem Ausführungsbeispiel ist auf der Leiterplatte 236 eine Ermittlungseinrichtung auf, die ausgebildet ist, um unter Verwendung zumindest eines Signals der Empfangseinrichtung 234 einen die Dampfkonzentration repräsentierenden Wert zu ermitteln.

Die Sendeeinrichtung 232 ist ausgebildet, um zumindest einen eine erste Wellenlänge aufweisenden ersten elektromagnetischen Strahl auszusenden. Gemäß einem Ausführungsbeispiel ist die Sendeeinrichtung 232 ausgebildet, um ferner einen zweiten elektromagnetischen Strahl bereitzustellen, der eine sich von der ersten Wellenlänge unterscheidende zweite Wellenlänge aufweist. Beispielsweise wird der Strahl oder werden die Strahlen über die mit der Sendeeinrichtung 232 gekoppelten Lichtfaserdurchführungen 230 zu einer in dem Sensorkopf 114 angeordneten Messkammer geleitet.

Die Empfangseinrichtung 234 ist ausgebildet ist, um eine Intensität des oder der von der Sendeeinrichtung 232 ausgesendeten Strahlen nach Passieren des Sensorkopfes 114 zu erfassen und für jede erfasste Intensität ein Intensitätssignal bereitzustellen.

Gemäß einem Ausführungsbeispiel sind in den Sensorkopf 114 Faserdurchführungen 230 mit eingeklebten oder eingegossenen Lichtfasern mit einen fixen Abstand eingeschraubt. Alternativ können die Lichtfaser statt geklebt oder vergossen zu sein, mit einem PTFE/Silikon-Einsatz in dem Sensorkopf 114 druckdicht befestigt sein.

Am Ende einer Faserdurchführung 230 befindet sich gemäß einem Ausführungsbeispiel die eine Optik mit LEDs umfassende Sendeeinrichtung 232. In der Sendeeinrichtung 232 werden Lichtwellen generiert und über die Faserdurchführungen 230 durch den Sensorkopf 114 befördert. Am Ende der anderen der Faserdurchführungen 230 befindet sich die Empfangseinrichtung 234 als Empfänger der Lichtwellen.

Gemäß einem Ausführungsbeispiel befinden sich am Sensorkopf 114 zwei Magnetventile, das Dampfventil 224 und das Belüftungsventil 226. Eine Belüftung erfolgt gemäß diesem Ausführungsbeispiel über den separaten Anschluss 228 mit Reinluftfilter. Um den Temperaturverlust zu minimieren wird der Sensorkopf unter Verwendung der Isolierung 222 isoliert.

Das Gehäuse für die Elektronik und Optik der Betriebseinrichtung 116 ist gemäß diesem Ausführungsbeispiel um den Sensorkopf 114 montiert. Im Gehäuse wird die Leiterplatte 236, beispielsweise eine PCB oder eine Elektronikplatine verbaut, die alle notwendigen Elektrokomponenten beinhaltet. Es ist möglich, falls notwendig das Gehäuse mit einer aktiven Kühlung 238, beispielsweise wasser- oder luftbasiert auszustatten.

Im Inneren des Sensorkopfes 114 ist gemäß einem Ausführungsbeispiel ein Temperaturfühler platziert. Ein von dem Temperaturfühler bereitgestelltes Temperatursignal ermöglicht eine durchgehende Überwachung der Temperatur des Sensorkopfes 114. Optional ist in dem Inneren des Sensorkopfes 114 ferner ein Drucksensor platziert.

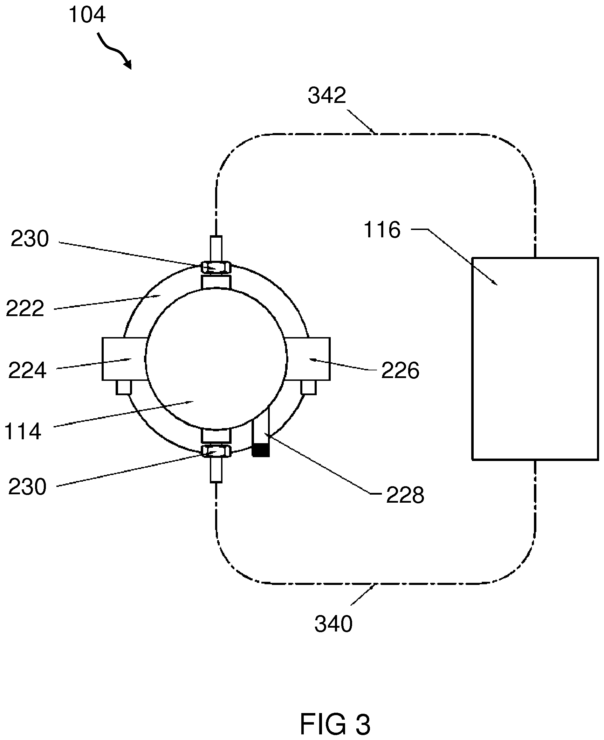

Figur 3 zeigt eine Vorrichtung 104 zum Erfassen einer Dampfkonzentration gemäß einem Ausführungsbeispiel. Dabei kann es sich um ein Ausführungsbeispiel der anhand von Figur 1 beschriebenen Vorrichtung handeln. Die Vorrichtung 104 umfasst den Sensorkopf 114 und die Betriebseinrichtung 116.

Die Vorrichtung 104 entspricht der anhand von Figur 3 beschriebenen Vorrichtung, mit dem Unterschied, dass das Gehäuse der Betriebseinrichtung 116 mit der Optik/Elektronik entfernt vom Sensorkopf 114 aufgebaut ist. Beispielhaft ist die Verbindung zwischen Sensorkopf 114 und dem Gehäuse der Betriebseinrichtung 116 über Lichtleiter, hier Lichtfasern 340, 342 realisiert.

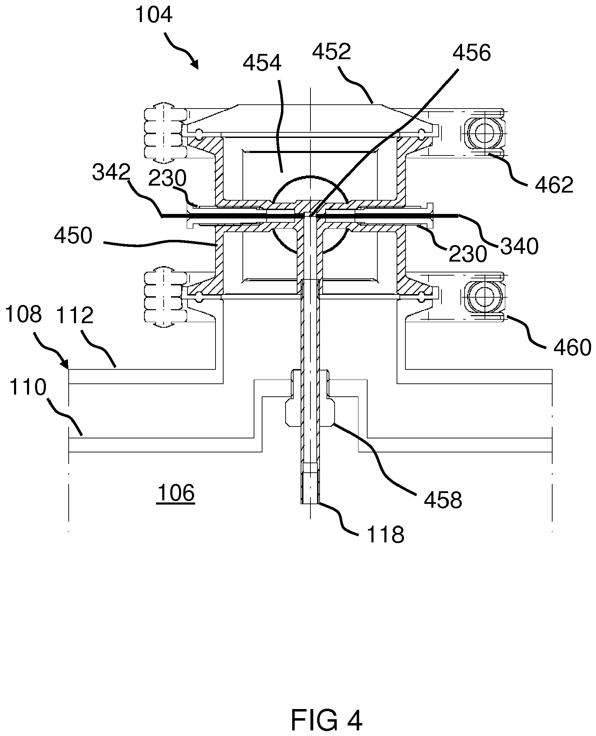

Figur 4 zeigt eine Vorrichtung 104 zum Erfassen einer Dampfkonzentration gemäß einem Ausführungsbeispiel. Dabei kann es sich um ein Ausführungsbeispiel der anhand von Figur 1 beschriebenen Vorrichtung handeln. Die Vorrichtung 104 ist an dem Doppelmantel 108 des Sterilisators befestigt. Dabei deckt der Sensorkopf der Vorrichtung 104 eine sich in der äußeren Wand 110 befindliche Mantelöffnung ab.

Das Gehäuse der Vorrichtung 104 setzt sich aus einem umlaufenden Wandelement 450 und einem Deckel 452 zusammen. Der Deckel 452 verschließt eine obere Öffnung des Wandelements 450, sodass ein Hohlraum 454 von dem Wandelement 450 und dem Deckel 452 umschlossen wird. Dem Deckel 452 gegenüberliegend weist das Wandelement 450 eine Gehäuseöffnung auf, die gegenüberliegend zu der Mantelöffnung angeordnet ist. Über die Gehäuseöffnung und die Mantelöffnung ist der Hohlraum 454 fluidisch mit einem Innenbereich des Doppelmantels 108 verbunden. Auf diese Weise kann in dem Doppelmantel 108 strömender Dampf in den Hohlraum 454 gelangen.

In dem Hohlraum 454 ist eine Messkammer 456 angeordnet. Die Messkammer 456 ist über eine Verbindungsleitung 118 fluidisch mit dem Sterilisationsraum 106 verbunden. Auf diese Weise kann sich innerhalb des Sterilisationsraums 106 befindlicher Dampf in die Messkammer 456 gelangen. Dazu ist die Verbindungsleitung 118 durch die Gehäuseöffnung und die Mantelöffnung sowie durch eine Öffnung der inneren Wand 110 geführt. Die innere Wand 110 ist gegenüber der Verbindungsleitung 118 abgedichtet, beispielsweise mit einer Verschraubung 458.

An die Messkammer 456 schließen zwei Lichtfaserdurchführungen 230 an. Durch eine der Lichtfaserdurchführung 230 ist eine erste Lichtleiteinrichtung, hier eine erste Lichtfaser 340 und durch die andere der Lichtfaserdurchführungen 230 ist eine zweite Lichtleiteinrichtung, hier eine zweite Lichtfaser 342 geführt. Durch die erste Lichtfaser 340 wird zumindest ein erster elektromagnetischer Strahls in ein von der Messkammer 456 umfasstes Messlumen eingeleitet. Durch die zweite Lichtfaser 342 wird zumindest ein Anteil des zumindest einen ersten elektromagnetischen Strahls nach Durchdringen des Messlumens aus der Messkammer 456 herausgeleitet.

Gemäß diesem Ausführungsbeispiel weist das Wandelement 450 einen die Gehäuseöffnung umschließenden Gehäuseflansch auf, der auf einen entsprechenden Mantelflansch der äußeren Wand 112 aufgesetzt ist. Gehäuseflansch und Mantelflansch sind unter Verwendung einer Verbindungseinrichtung 460, beispielsweise einer sogenannten Tri-Clamp-Verbindung fluiddicht miteinander verbunden. Gemäß einem Ausführungsbeispiel ist ein Dichtring zwischen dem Gehäuseflansch und dem Mantelflansch angeordnet.

Gemäß diesem Ausführungsbeispiel sind Wandelement 450 und Deckel 452 über eine weitere Verbindungseinrichtung 462, beispielsweise einer sogenannten Tri-Clamp-Verbindung, fluiddicht miteinander verbunden. Gemäß einem alternativen Ausführungsbeispiel ist das Gehäuse einstückig ausgeführt.

Gemäß einem Ausführungsbeispiel zeigt Figur 4 die Vorrichtung 104, beispielsweise in Form eines 4D-Sensors, in der Schnittebene der Lichtfaserdurchführungen 230. Der 4D-Sensor wird z.B. mit Hilfe der Verbindungseinrichtung 460 in Form einer Tri-Clamp-Verbindung am Doppelmantel 108 beispielsweise eines Groß-Dampf-Sterilisators (DGS) befestigt. Das in der Messkammer 456 angeordnete Messlumen des Sensors wird über die Verbindungsleitung 118 mit dem Sterilisationsraum 106 verbunden.

Der Sensorkopf hat im Inneren den Hohlraum 454, der mit dem Doppelmantel 108 des Sterilisators verbunden ist. So ist gewährleistet, das der Sensorkopf immer mit Doppelmanteldampf durchströmt wird und dadurch beheizt wird. Das Beheizen ist sinnvoll, damit die Temperatur im Messlumen der Messkammer 456 immer über der Sattdampftemperatur gehalten wird und damit Kondensierung im Messlumen verhindert wird. Kondensat im Messlumen würde eine aussagekräftige Messung der Sattdampfkonzentration verhindern.

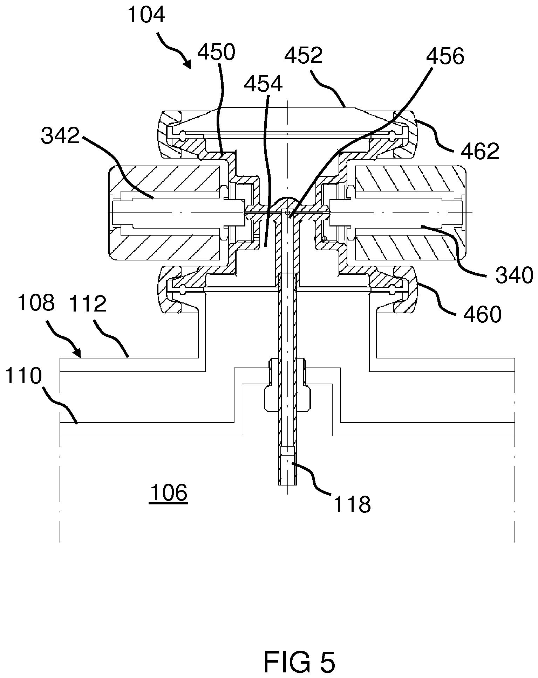

Figur 5 zeigt eine Vorrichtung 104 zum Erfassen einer Dampfkonzentration gemäß einem Ausführungsbeispiel. Dabei kann es sich um eine weitere Schnittdarstellung der anhand von Figur 4 beschriebenen Vorrichtung handeln. Dabei ist eine Schnittebene durch die Ventile 224, 226 in Form des bereits anhand von Figur 2 genannten Dampfventils 224 und des Belüftungsventils 226 gezeigt.

Gemäß einem Ausführungsbeispiel sind die Ventile 224, 226 an einer Außenseite des Wandelements 450 angeordnet. Das Wandelement 450 weist gemäß einem Ausführungsbeispiel Einbuchtungen auf, in die die Ventile 224, 226 eingepasst werden können.

Das Dampfventil 224 ist ausgangsseitig mit einer Dampfventilöffnung der Messkammer 456 verbunden. Wird das Dampfventil 224 geöffnet, so kann Kalibrierdampf in die Messkammer 456 einströmen. Dazu ist das Dampfventil 224 beispielhaft eingangsseitig mit dem Hohlraum 454 verbunden.

Gemäß einem Ausführungsbeispiel hat das Dampfventil 224 die Funktion, das Dampf aus dem Doppelmantel 108 in das Messlumen der Messkammer 456 zur Kalibrierungszwecken eingeblasen wird. Alternativ zur Verwendung des Doppelmanteldampfes kann der Dampf für das Kalibrieren zusätzlich oder alternative aus einer äußeren Quelle, beispielsweise einer extern zu dem Sterilisator angeordneten Dampfbereitstellungseinrichtung, stammen.

Das Belüftungsventil 340 ist ausgangsseitig mit einer Belüftungsöffnung der Messkammer 456 verbunden. Wird das Belüftungsventil 340 geöffnet so kann Reinluft, beispielsweise aus der Umgebung der Vorrichtung 104, in die Messkammer 456 einströmen.

Gemäß einem Ausführungsbeispiel wird über das Belüftungsventil 340 Luft über einen Reinluftfilter in das Messlumen eingelassen. Das Belüften wird zwecks Konditionierung und Trocknung des Messlumen durchgeführt.

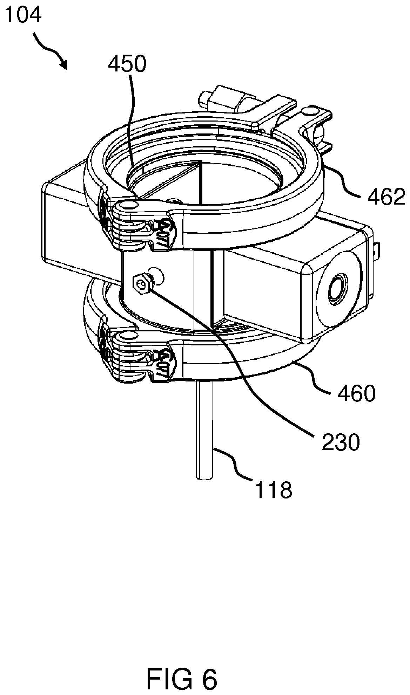

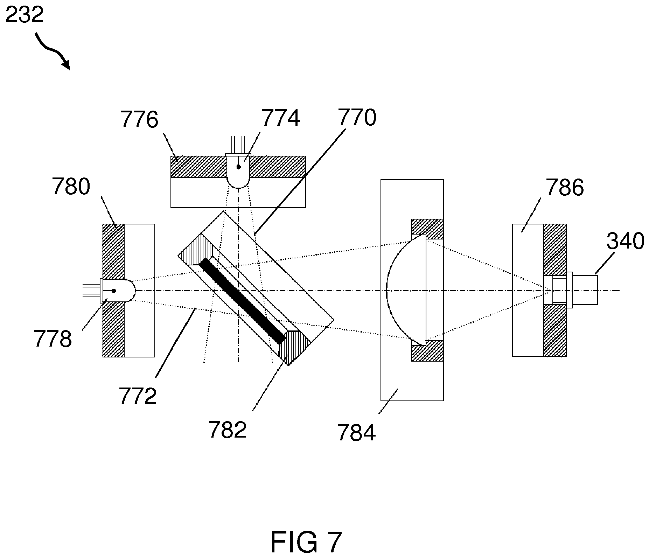

Figur 6 zeigt eine dreidimensionale Darstellung einer Vorrichtung 104 zum Erfassen einer Dampfkonzentration gemäß einem Ausführungsbeispiel. Dabei kann es sich um eine Darstellung der anhand von Figur 4 und 5 beschriebenen Vorrichtung handeln Figur 7 zeigt eine Sendeeinrichtung 232 gemäß einem Ausführungsbeispiel. Die Sendeeinrichtung 232 kann als Teil oder als Ergänzung einer anhand der vorangegangenen Figuren beschriebenen Vorrichtung zum Erfassen einer Dampfkonzentration verwendet werden. Gemäß diesem Ausführungsbeispiel ist die Sendeeinrichtung 232 ausgebildet, um einen ersten elektromagnetischen Strahl 770 in Form eines erste Lichtstrahls und einen zweiten elektromagnetischen Strahl 772 in Form eines zweiten Lichtstrahls bereitzustellen.

Gemäß diesem Ausführungsbeispiel umfasst die Sendeeinrichtung 232 zum Erzeugen des ersten Strahls 770 eine erste Leuchtdiode 774, die von einer ersten Leuchtdiodenhalterung 776 gehalten wird. Entsprechend umfasst die Sendeeinrichtung 232 zum Erzeugen des zweiten Strahls 772 eine zweite Leuchtdiode 778, die von einer zweiten Leuchtdiodenhalterung 780 gehalten wird. Die Leuchtdioden 774, 778 sind rechtwinklig zueinander angeordnet. Unter Verwendung eines Reflektors 782 wird der erste Strahl 770 in eine Ausbreitungsrichtung des zweiten Strahls 772 umgelenkt. Unter Verwendung einer Linse 784 werden die Strahlen 770, 772 auf eine von einem Lichtfaseranschluss 786 gehaltene Lichtfaser 340 gebündelt. Über die Lichtfaser 340 können die Strahlen 770, 772 zu der Messkammer der Vorrichtung zum Erfassen einer Dampfkonzentration geleitet werden.

Die Optik einer Vorrichtung zum Erfassen einer Dampfkonzentration, beispielsweise eines 4D-Sensors, wird gemäß einem Ausführungsbeispiel so aufgebaut, dass das Licht der ersten Leuchtdiode 774 und der zweiten Leuchtdiode 778 mit Hilfe der Linse 784 gebündelt und direkt auf die Lichtfaser 340 konzentrier wird. Damit beide Leuchtdioden 774, 778 einen Fokuspunkt haben, wird das Licht der ersten Leuchtdiode 774 mithilfe des Reflektors 782 um 90° gespiegelt.

Es werden gemäß einem Ausführungsbeispiel Leuchtdioden 774, 778 mit zwei verschiedenen Wellenlängen Leuchtdioden 774, 778, beispielhaft eine mit 1300nm die andere mit 1450nm. Es können alternative Leuchtdioden 774, 778 eingesetzt werden mit anderen Wellenlängen die sich immer noch im angestrebten Absorption-Bereich bewegen.

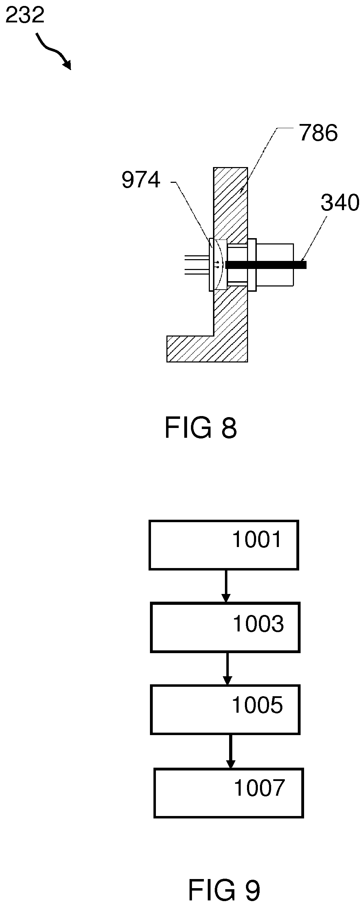

Figur 8 zeigt eine Sendeeinrichtung 232 gemäß einem Ausführungsbeispiel. Die Sendeeinrichtung 232 kann als Teil oder als Ergänzung einer anhand der vorangegangenen Figuren beschriebenen Vorrichtung zum Erfassen einer Dampfkonzentration verwendet werden. Im Unterschied zu dem anhand von Figur 7 beschriebenen Ausführungsbeispiel wird eine Doppel-Leuchtdiode 974 eingesetzt, um zwei Strahlen unterschiedlicher Wellenlängen bereitzustellen. Gemäß einem Ausführungsbeispiel ist die Doppel-Leuchtdiode 974 klein genug, um direkt an die Lichtfaser 340 angebracht zu werden. Dazu kann die Doppel-Leuchtdiode 974 an dem Lichtfaseranschluss 786 befestigt werden. Diese Konstruktion hat den Vorteil, das kein Reflektor und auch keine Linse benötigt wird.

Figur 9 zeigt ein Ablaufdiagramm eines Verfahrens zur Kalibrierung einer Vorrichtung zum Erfassen einer Dampfkonzentration gemäß einem Ausführungsbeispiel. Das Verfahren kann verwendet werden, um eine anhand der vorangegangenen Figuren beschriebenen Vorrichtung zum Erfassen einer Dampfkonzentration zu kalibrieren.

In einem Schritt 1001 wird Reinluft in die Messkammer der Vorrichtung eingelassen. Dazu wird beispielsweise das anhand von Figur 5 beschriene Belüftungsventil geöffnet. In einem Schritt 1003 wird zumindest ein Nullpunktwert unter Verwendung einer Intensität des zumindest einen ersten Strahls nach Einleitung des ersten Strahls in die die Reinluft umfassende Messkammer erfasst und bereitgestellt. Die Intensität wird beispielsweise unter Verwendung der anhand von Figur 2 gezeigten Empfangseinrichtung erfasst. In einem Schritt 1005 wird Kalibrierdampf in die Messkammer eingelassen. Dazu wird beispielsweise das anhand von Figur 5 beschriene Dampfventil geöffnet. In einem Schritt 1007 wird zumindest ein Kalibrierwert unter Verwendung einer Intensität des zumindest einen ersten Strahls nach Einleitung des ersten Strahls in die den Kalibrierdampf umfassende Messkammer. Die Intensität wird beispielsweise wiederum unter Verwendung der anhand von Figur 2 gezeigten Empfangseinrichtung erfasst.

Gemäß einem Ausführungsbeispiel werden zur Ansteuerung der Ventile erforderlichen Steuersignale von einer Steuervorrichtung bereitgestellt.

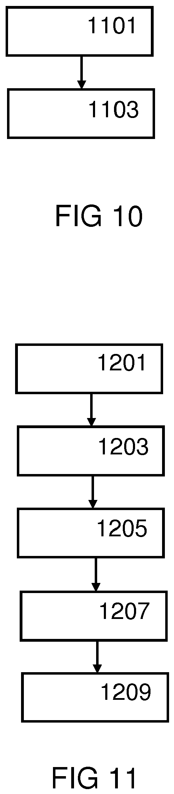

Figur 10 zeigt ein Ablaufdiagramm eines Verfahrens zur Ermittlung einer Dampfkonzentration in einem Sterilisator unter Verwendung einer Vorrichtung zum Erfassen einer Dampfkonzentration gemäß einem Ausführungsbeispiel.

Das Verfahren kann verwendet werden, um unter Verwendung einer anhand der vorangegangenen Figuren beschriebenen Vorrichtung zum Erfassen einer Dampfkonzentration zu verwenden.

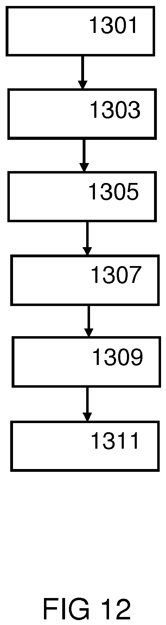

In einem Schritt 1101 wird einer Intensität des zumindest einen Anteils des zumindest einen ersten Strahls erfasst, während die Messkammer über die Verbindungsleitung fluidisch mit dem Sterilisationsraum verbunden ist. Dabei wird die Intensität gemäß einem Ausführungsbeispiel erfasst, wenn sich die Dampfkonzentration in der Messkammer an die Dampfkonzentration in dem Sterilisationsraum angepasst hat. Die Intensität wird beispielsweise unter Verwendung der anhand von Figur 2 gezeigten Empfangseinrichtung erfasst. In einem Schritt 1103 wird ein die Dampfkonzentration repräsentierender Wert unter Verwendung der im Schritt 1101 erfassten Intensität, eines Nullpunktwerts und eines Kalibrierwertes bestimmt. Dabei wurden der Nullpunktwert und der Kalibrierwert gemäß einem Ausführungsbeispiel gemäß dem anhand von Figur 9 oder den anhand der Figuren 11 und 12 beschriebenen Verfahren bestimmt.

Anhand der Figuren 11 und 12 wird eine Funktion anhand der vorangegangenen Figuren beschriebenen Vorrichtung zum Erfassen einer Dampfkonzentration anhand eines Ausführungsbeispiels detailliert beschrieben. Dabei wird die Vorrichtung beispielhaft als Sensor und insbesondere als 4D-Sensor bezeichnet.

Die Hauptfunktion des 4D-Sensors ist gemäß einem Ausführungsbeispiel die Messung der Konzentration von Sattdampf. Durch Lichtfasern wird Licht von zwei verschiedenen Leuchtdioden durch das Messlumen geleitet. Wenn Dampf vorhanden ist, wird das Licht der ersten Leuchtdiode absorbiert. Die zweite Leuchtdiode dient als Referenzleuchtdiode. Das Licht der Leuchtdioden wird von einem Empfänger erfasst und ein Signal wird dann beispielhaft über folgende Formel berechnet:

$$V_1 = I_1 \times \eta_{LED2} \times S(\lambda_1) \times [1 - \rho \times d \times f(\lambda_1)] \times \eta_{DET}(\lambda_1) \times G_{ADC}$$

$$V_2 = I_2 \times \eta_{LED2} \times S(\lambda_2) \times [1 - \rho \times d \times f(\lambda_2)] \times \eta_{DET}(\lambda_2) \times G_{ADC}$$

Legende:

| | |
|---|---|
| $V1$, $V2$: | Signale der Leuchtdioden LED1, LED2 |
| $I_1$, $I_2$: | LED Effizienz |
| $S(\lambda_1, \lambda_2)$: | Fasersystemübertragung |
| $\rho$: | Dampfdichte |
| $d$: | Lichtweg |
| $f(\lambda_1, \lambda_2)$: | spezifische Absorption |
| $\eta_{DET}(\lambda_1, \lambda_2)$: | Detektoreffizienz |
| $G_{ADC}$: | ADC Verstärkung |

Da das Signal der Absorption-LED bei vorhandenen Dampf absorbiert wird und das der Referenz-LED nicht, wird das Delta der beiden Signale berechnet und für die Auswertung und weitere Verarbeitung genutzt.

$$\Delta V = V_\rho = V_1 - V_2$$

Das einzigartige an dieser Ausführung des Sensors ist, dass die Möglichkeit besteht den Sensor auch direkt im Einsatz zu Konditionieren und zu Kalibrieren.

Die Konditionierung bezieht sich auf das Einstellen des Nullpunktes/Nullwertes des Sensors. Als Null-Wert wird der Punkt erfasst bei den sich im Messlumen kein Dampf und keine Feuchte befindet. Um zu erreichen, dass der Sensor im Messlumen komplett von Dampf/Feuchte befreit wird, ist konstruktiv ein Anschluss vorgesehen der über ein Magnetventil Reinluft in das Messlumen einlassen kann und damit den Dampf und die Feuchte entfernt.

Figur 11 zeigt ein Ablaufdiagramm eines Verfahrens zur Konditionierung einer Vorrichtung zum Erfassen einer Dampfkonzentration gemäß einem Ausführungsbeispiel. Das Verfahren kann verwendet werden, um eine anhand der vorangegangenen Figuren beschriebenen Vorrichtung zum Erfassen einer Dampfkonzentration zu konditionieren.

Die Konditionierung wird in der Trocknungsphase durchgeführt. Es können auch andere Zeitpunkte festgelegt werden in denen die Konditionierung gemacht wird.

Der Konditionierungsprozess läuft wie folgt ab:

In einem Schritt 1201 wird das Belüftungsventil der Vorrichtung geöffnet. In einem Schritt 1203 wird das Belüftungsventil für einen Zeitraum X geöffnet gelassen. Nachdem das Messlumen während des Schritts 1203 mit einer ausreichenden Menge Luft durchblassen wurde, der Dampf entfernt und das Messlumen getrocknet wurde, wird das Belüftungsventil in einem Schritt 1205 geschlossen. Wenn sich nach dem Schließen des Belüftungsventils innerhalb einer festgelegten Zeitspane das Signal, also beispielsweise die erfassten Intensitäten der Strahlen, nicht ändert, werden die Werte $V_1^0$ - ($V_1$ bei 0% Dampf) und $V_2^0$-($V_2$ bei 0% Dampf) in einem Schritt 1207 gespeichert. Falls nötig kann die Konditionierung bei offenen Belüftungsventil vollzogen werden, wenn sich das Sensorsignal innerhalb einer festgelegt Zeitspane nicht ändert. In einem Schritt 1209 wird der Null-Wert gemäß den neuen Konditionierungswerten aus dem Schritt 1205 gesetzt

Der Ablauf der Konditionierung ist in dem in Figur 11 gezeigten Flussdiagramm abgebildet.

Figur 12 zeigt ein Ablaufdiagramm eines Verfahrens zur Kalibrierung einer Vorrichtung zum Erfassen einer Dampfkonzentration gemäß einem Ausführungsbeispiel. Das Verfahren kann verwendet werden, um eine anhand der vorangegangenen Figuren beschriebenen Vorrichtung zum Erfassen einer Dampfkonzentration zu kalibrieren.

Die Kalibrierung wird während eines speziell dafür gemachten Sterilisations-Zyklus durchgeführt. Die Kalibration wird gemäß einem Ausführungsbeispiel wie folgt durchgeführt:

In einem Schritt 1301 wird der Kalibrierungszyklus gestartet. Am Anfang des Kalibrierungszyklus wird immer eine Konditionierung 1303 durchgeführt, beispielsweise durch Ausführen der anhand von Figur 12 gezeigten Schritte. Nach Eintritt in die Haltephase wird in einem Schritt 1305 das Dampfventil geöffnet. In einem Schritt 1307 wird das Dampfventil für einen definierten Zeitraum offengelassen bis der Inhalt des Messlumen ausreichend mit Dampf aus dem Doppelmantel durchströmt wurde. Es kann alternative auch Dampf aus anderen Quellen benutzt werden In einem Schritt 1309 wird das Dampfventil geschlossen. In einem Schritt 1311 wird das Messsignal erfasst. Wenn das Messsignal für einen festgelegten Zeitraum stabil bleibt, wird der Wert $V\rho$ erfasst und gespeichert.

Der Ablauf der Kalibrierung ist in dem in Figur 12 gezeigten Flussdiagramm abgebildet.

Im Folgenden wird eine Zusammenfassung des Verfahrens im Erfassungssystem gegeben:

Während des anhand von Figur 11 beschriebenen Verfahrens der Konditionierung befindet sich der Sensor in einem Zustand, bei dem 0 % Dampf vorhanden ist. Es werden als Aktion die Werte $V_1^0$ und $V_2^0$ gespeichert.

Während des anhand von Figur 12 beschriebenen Verfahrens der Kalibrierung befindet sich der Sensor in einem Zustand, bei dem 100 % Dampf vorhanden ist. Es wird als Aktion der Wert $V\rho(100$ %$)$ gespeichert.

Während des beispielsweise anhand von Figur 10 beschriebenen Verfahrens des Messens befindet sich der Sensor in einem Zustand, bei dem der Dampfanteil in dem Messlumen zwischen 0 und 100 % liegt. Es wird als Aktion eine lineare Berechnung zur Bestimmung der Dampfkonzentration durchgeführt: $(V\rho$-$V\rho(0))$ / $(V\rho(100)$-$V\rho(0))$.

**Patentansprüche**

1. Vorrichtung (104) zum Erfassen einer Dampfkonzentration in einem Sterilisator (102) mit einem einen Sterilisationsraum (106) umschließenden Doppelmantel (108), wobei der Doppelmantel (108) eine innere Wand (110) und eine äußere Wand (112) mit einer Mantelöffnung aufweist, und wobei die Vorrichtung (104) die folgenden Merkmale aufweist:

    ein Gehäuse (220), das mit der äußeren Wand (112) des Sterilisators (102) koppelbar ist und eine Gehäuseöffnung aufweist, über die ein von dem Gehäuse (220) umschlossener Hohlraum (454) fluidisch mit der Mantelöffnung verbindbar ist;
    eine Messkammer (456), die in dem Hohlraum (454) angeordnet ist;
    eine Verbindungsleitung (118), die mit der Messkammer (456) gekoppelt ist, um die Messkammer (456) fluidisch mit einem Sterilisationsraum (106) des Sterilisators (102) zu verbinden;
    eine erste Lichtleiteinrichtung (340) zum Einleiten zumindest eines ersten elektromagnetischen Strahls (770) in ein von der Messkammer (456) umfasstes Messlumen; und
    eine zweite Lichtleiteinrichtung (342) zum Ausleiten zumindest eines Anteils des zumindest einen ersten elektromagnetischen Strahls (770) aus dem Messlumen.

2. Vorrichtung (104) gemäß Anspruch 1, bei der das Gehäuse (220) einen die Gehäuseöffnung umschließenden Gehäuseflansch zum mechanischen Verbinden des Gehäuses (220) mit der äußeren Wand (112) des Doppelmantels (108) aufweist.

3. Vorrichtung (104) gemäß einem der vorangegangenen Ansprüche, mit einem Dampfventil, das ausgangsseitig mit einer Dampfventilöffnung der Messkammer (456) verbunden ist, und ausgebildet ist, um Kalibrierdampf in die Messkammer (456) einzulassen.

4. Vorrichtung (104) gemäß Anspruch 4, bei der das Dampfventil eingangsseitig mit dem Hohlraum (454) verbunden ist.

5. Vorrichtung (104) gemäß einem der vorangegangenen Ansprüche, mit einem Belüftungsventil, das ausgangsseitig mit einer Belüftungsöffnung der Messkammer (456) verbunden ist, und ausgebildet ist, um Reinluft in die Messkammer (456) einzulassen.

6. Vorrichtung (104) gemäß einem der vorangegangenen Ansprüche, bei der die erste Lichtleiteinrichtung (340) einen durch das Gehäuse (220) zu einer ersten Lichtleiteröffnung der Messkammer (456) führenden ersten Lichtleiter und/oder die zweite Lichtleiteinrichtung (342) einen durch das Gehäuse (220) zu einer zweiten Lichtleiteröffnung der Messkammer (456) führenden zweiten Lichtleiter aufweist.

7. Vorrichtung (104) gemäß einem der vorangegangenen Ansprüche, mit einer Sendeeinrichtung, die ausgebildet ist, um den eine erste Wellenlänge aufweisenden ersten elektromagnetischen Strahl (770) und einen eine sich von der ersten Wellenlänge unterscheidende zweite Wellenlänge aufweisenden zweiten elektromagnetischen Strahl (772) bereitzustellen und in die erste Lichtleiteinrichtung (340) einzukoppeln.

8. Vorrichtung (104) gemäß Anspruch 7, bei der die Sendeeinrichtung eine erste Leuchtdiode zum Aussenden des ersten elektromagnetischen Strahls (770) und eine zweite Leuchtdiode zum Aussenden des zweiten elektromagnetischen Strahls (772) aufweist.

9. Vorrichtung (104) gemäß einem der vorangegangenen Ansprüche, mit einer Empfangseinrichtung, die mit der zweiten Lichtleiteinrichtung (342) gekoppelt und ausgebildet ist, um ein eine Intensität des zumindest einen Anteils des ersten elektromagnetischen Strahls (770) repräsentierendes erstes Intensitätssignal bereitzustellen, und ausgebildet ist, um ein eine Intensität zumindest eines Anteils eines zweiten elektromagnetischen (772) repräsentierendes zweites Intensitätssignal bereitzustellen.

10. Vorrichtung (104) gemäß Anspruch 9, mit einer Ermittlungseinrichtung, die ausgebildet ist, um unter Verwendung des ersten Intensitätssignals und des zweiten Intensitätssignals einen die Dampfkonzentration repräsentierenden Wert zu ermitteln.

11. Sterilisatorvorrichtung (100) mit folgenden Merkmalen:

    einem Sterilisator (102) mit einem einen Sterilisationsraum (106) umschließenden Doppelmantel (108), wobei der Doppelmantel (108) eine innere Wand (110) und eine äußere Wand (112) mit einer Mantelöffnung aufweist; und
    einer Vorrichtung (104) gemäß einem der vorangegangenen Ansprüche zum Erfassen einer Dampfkonzentration in dem Sterilisator (102).

12. Sterilisatorvorrichtung (100) gemäß Anspruch 11, bei der der Doppelmantel (108) im Betrieb der Sterilisatorvorrichtung (100) von Dampf durchströmt wird.

13. Verfahren zur Kalibrierung einer Vorrichtung (104) gemäß einem der vorangegangenen Ansprüche, wobei das Verfahren die folgenden Schritte umfasst:

    Einlassen (1001) von Reinluft in die Messkammer (456);
    Bestimmen (1003) eines Nullpunktwerts unter Verwendung einer Intensität des zumindest einen ersten Strahls (770) nach Einleitung des ersten Strahls (770) in die die Reinluft umfassende Messkammer (456);
    Einlassen (1005) von Kalibrierdampf in die Messkammer (456); und
    Bestimmen (1007) eines Kalibrierwertes unter Verwendung einer Intensität des zumindest einen ersten Strahls (770) nach Einleitung des ersten Strahls (770) in die den Kalibrierdampf umfassende Messkammer (456).

14. Verfahren zur Ermittlung einer Dampfkonzentration in einem Sterilisator (102) unter Verwendung einer Vorrichtung (104) gemäß einem der vorangegangenen Ansprüche, wobei das Verfahren die folgenden Schritte umfasst:

Bestimmen (1101) einer Intensität des zumindest einen Anteils des zumindest einen ersten Strahls (770), während die Messkammer (456) über die Verbindungsleitung (118) fluidisch mit dem Sterilisationsraum (106) verbunden ist; und

Bestimmen (1103) eines die Dampfkonzentration repräsentierenden Werts unter Verwendung der Intensität, eines Nullpunktwerts und eines Kalibrierwertes, wobei der Nullpunktwert und der Kalibrierwert gemäß dem Verfahren gemäß Anspruch 13 bestimmte Werte repräsentieren.

100

104

116

114

102

106

108

118

112

110

FIG 1

**FIG 2**

FIG 3

FIG 4

FIG 5

104
450
462
460
230
118

FIG 6

232

FIG 7

232

786

974

340

## FIG 8

1001

1003

1005

1007

## FIG 9

```
┌─────────────────────┐
│              1101   │
└─────────────────────┘
           │
           ▼
┌─────────────────────┐
│              1103   │
└─────────────────────┘
```

# FIG 10

```
┌─────────────────────┐
│              1201   │
└─────────────────────┘
           │
           ▼
┌─────────────────────┐
│              1203   │
└─────────────────────┘
           │
           ▼
┌─────────────────────┐
│              1205   │
└─────────────────────┘
           │
           ▼
┌─────────────────────┐
│              1207   │
└─────────────────────┘
           │
           ▼
┌─────────────────────┐
│              1209   │
└─────────────────────┘
```

# FIG 11

```
┌──────────────────┐
│            1301  │
└──────────────────┘
          │
          ▼
┌──────────────────┐
│            1303  │
└──────────────────┘
          │
          ▼
┌──────────────────┐
│            1305  │
└──────────────────┘
          │
          ▼
┌──────────────────┐
│            1307  │
└──────────────────┘
          │
          ▼
┌──────────────────┐
│            1309  │
└──────────────────┘
          │
          ▼
┌──────────────────┐
│            1311  │
└──────────────────┘
```

FIG 12

Europäisches
Patentamt

European
Patent Office

Office européen
des brevets

# EUROPÄISCHER RECHERCHENBERICHT

Nummer der Anmeldung

EP 18 19 3917

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 20 2014 009495 U1 (HOLZNER MEDIZINTECHNIK GMBH [DE]) 5. Februar 2015 (2015-02-05) | 1,2,6, 10-12 | INV. G01N21/3504 A61L2/07 |
| Y | * Absätze [0002], [0018], [0019] * <br> * Abbildungen 1-3 * <br> ----- | 3-5 | A61L2/28 G01N21/31 |
| X | Jpcm Doornmalen - Gomez Hoyos ET AL: "Surface steam sterilization : steam penetration in narrow channels", , 1. Januar 2013 (2013-01-01), XP055388492, DOI: 10.6100/IR758412 ISBN: 978-90-38-63442-5 Gefunden im Internet: URL:https://pure.tue.nl/ws/files/3573650/758412.pdf | 1,2,6-14 | |
| Y | * Seiten 14,15 * <br> * Seiten 59,60 * <br> * Abbildungen 2.1,6.4 * <br> ----- | 3-5 | |
| X | US 5 892 229 A (CROZIER DAVID [US] ET AL) 6. April 1999 (1999-04-06) * Spalte 1, Zeilen 6-9 * * Spalte 2, Zeilen 33-43 * * Spalte 4, Zeilen 41-44 * * Abbildungen 1,3,10,11 * ----- | 1,6-14 | RECHERCHIERTE SACHGEBIETE (IPC) G01N A61L |
| X | EP 0 639 987 B1 (UNIV WALES MEDICINE [GB]) 29. August 2001 (2001-08-29) * Absätze [0001], [0049], [0054] * * Abbildungen 1,8 * ----- | 1,2,11, 12 | |
| Y | US 6 512 230 B1 (VON LERBER TUOMO ANTERO [FI]) 28. Januar 2003 (2003-01-28) * Spalte 11, Zeilen 40-46 * ----- | 3-5 | |
| | -/-- | | |

Der vorliegende Recherchenbericht wurde für alle Patentansprüche erstellt

| Recherchenort | Abschlußdatum der Recherche | Prüfer |
|---|---|---|
| München | 17. Dezember 2018 | Brauer, Jan |

EPO FORM 1503 03.82 (P04C03)

Europäisches
Patentamt

European
Patent Office

Office européen
des brevets

# EUROPÄISCHER RECHERCHENBERICHT

Nummer der Anmeldung

EP 18 19 3917

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2012/173562 A1 (TETRA LAVAL HOLDINGS & FINANCE [CH]; OMRANE ALAA [SE]) 20. Dezember 2012 (2012-12-20) * Seite 1, Zeilen 6-8 * * Seite 3, Absatz 5 * * Seite 5, Absätze 1,2 * * Seite 6, Absatz 2 * * Seite 9, Absatz 1 * * Seite 11, Absatz 1 * * Abbildung 4 *  ----- | 1,2,6-14 | |
| | | | **RECHERCHIERTE SACHGEBIETE (IPC)** |

Der vorliegende Recherchenbericht wurde für alle Patentansprüche erstellt

| Recherchenort | Abschlußdatum der Recherche | Prüfer |
|---|---|---|
| München | 17. Dezember 2018 | Brauer, Jan |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument
...........................................................................................
& : Mitglied der gleichen Patentfamilie, übereinstimmendes
Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 18 19 3917

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-12-2018

| Im Recherchenbericht angeführtes Patentdokument | Datum der Veröffentlichung | Mitglied(er) der Patentfamilie | | Datum der Veröffentlichung |
|---|---|---|---|---|
| DE 202014009495 U1 | 05-02-2015 | KEINE | | |
| US 5892229 A | 06-04-1999 | AU | 2552297 A | 12-11-1997 |
| | | EP | 0895589 A1 | 10-02-1999 |
| | | US | 5892229 A | 06-04-1999 |
| | | WO | 9740365 A1 | 30-10-1997 |
| EP 0639987 B1 | 29-08-2001 | AU | 6148094 A | 26-09-1994 |
| | | DE | 69428085 D1 | 04-10-2001 |
| | | DE | 69428085 T2 | 25-04-2002 |
| | | EP | 0639987 A1 | 01-03-1995 |
| | | WO | 9420150 A1 | 15-09-1994 |
| US 6512230 B1 | 28-01-2003 | EP | 1061355 A1 | 20-12-2000 |
| | | US | 6512230 B1 | 28-01-2003 |
| WO 2012173562 A1 | 20-12-2012 | KEINE | | |

EPO FORM P0461

Für nähere Einzelheiten zu diesem Anhang : siehe Amtsblatt des Europäischen Patentamts, Nr.12/82